# EUROPÄISCHE PATENTSCHRIFT

(11) **EP 1 779 791 B1**
(45) Veröffentlichungstag und Bekanntmachung des Hinweises auf die Patenterteilung: **29.04.2009**
(21) Anmeldenummer: 06017496.8
(22) Anmeldetag: 23.08.2006
(51) Int. Cl.: A61B 17/16, A61B 17/32, A61B 17/00, A61B 19/00

(54) **Facettengelenkfräser**
Facet joint milling tool
Fraise pour facette vertébrale

(30) Priorität: 26.10.2005 DE 202005016762 U
(43) Veröffentlichungstag der Anmeldung: 02.05.2007
(73) Patentinhaber: Joimax GmbH, 76227 Karlsruhe (DE)
(72) Erfinder: Notheis, Mathias, 63517 Rodenbach (DE); Ries, Wolfgang, 76351 Linkenheim (DE)
(74) Vertreter: Lempert, Jost

(56) Entgegenhaltungen:
- DE-T2- 69 917 683
- DE-U1- 8 806 721
- DE-U1-3202005 016 76
- JP-A- 2003 245 283
- US-A- 2 429 356
- US-A- 4 069 824
- US-A- 5 312 408
- US-A- 5 961 522
- US-B1- 6 322 564

## Beschreibung

Die Erfindung betrifft einen Facettengelenkfräser nach dem Oberbegriff des Anspruchs 1.

Ein gattungsgemäßer Facettengelenkfräser ist aus der DE 699 17 683 T2 bekannt. Der bekannte Fräser weist einen hohlen zylindrischen Schaft, an seinem rückwärtigen, proximalen Ende einen Handgriff und an seinem stirnseitigen Ende eine Zähnung auf.

Ein derartiger Fräser wird zum Ausfräsen von Wirbelbestandteilen im Bereich eines seitlichen Fortsatzes eines Wirbelsäulen-Wirbels eingesetzt, um einen postero-lateralen Zugang zu eingeklemmten Nervenwurzeln des zentralen Nervensystems zu schaffen. Durch diesen Zugang werden dann Nucleus-Pulposus-Gewebe und andere Gewebearten (Kapselgewebe, Narbengewebe, Annulus-Gewebe) entfernt, da diese auf die Nervenwurzeln drücken. Der benannte Fortsatz eines Wirbels bildet mit einem benachbarten Fortsatz eines benachbarten Wirbels das sogenannte Facettengelenk.

Die einen gattungsgemäßen Facettengelenkfräser einsetzende microinvasive Operationsmethode zur Dekompression von eingeklemmten Nervenwurzeln ist höchst erfolgreich. Allerdings hat sich herausgestellt, dass durch den Fräser abgetragenes Knochenmaterial nicht hinreichend aus dem Abtragbereich entfernt wird.

Die US 6,322,564 zeigt einen Femur-Fräser oder um ein System, um proximal eine Femuralprothese in einem im Femur ausgebildeten Kanal zu platzieren, wobei ein Teil dieses Systems eine Schneid- oder Fräseinrichtung ist, die mittels der Fräseinrichtung plangeschliffen und nur mit einer ringförmigen Referenznut versehen wird. Hierzu sieht der Fräskopf sich radial über nahezu seine gesamte Stirnseite erstreckende Schneiden vor, die über den Umfang hin verteilt sind, wobei etwas mittig der Schneiden in gleichem Radialabstand zu Symmetrieachse Zähne angeordnet sind, mittels derer eben gerade die kreisförmige Nut beim Drehen des Fräsers rotierbar ist. Die den sonstigen Zahnkörper jeweils überragende Mehrzahl von Zähnen des Femur-Fräsers sind keine Lippen und ihre Schneide verläuft radial bei exakt genau einem Umfangswinkelwert. Die Zahl dieser überragenden radialen Zähne entspricht der der Planierzähne.

Beim Gegenstand der US 2,429,356 ist ein drehbarer Fräser bzw. dessen Fräserkopf durch ein stationär gehaltenes Schutzschild teilweise überdeckt.

Beim Gegenstand der JP 2003-245283 handelt es sich um einen Drillbohrer mit schraubenförmiger Schneiden am Mantelumfang, der damit also zunächst keinen Schaft aufweist. Bei einem derartigen Bohrer ist zur Zentrierung an der Stirnseite eine Spitze vorhanden, die genau in einem auf der Achse liegenden Punkt ausläuft.

Der Erfindung liegt die Aufgabe zugrunde, einen gattungsgemäßen Facettengelenkfräser dahingehend weiterzuentwickeln, dass ein zuverlässiges Heraustragen von abgetragenem Knochenmaterial möglich ist, ohne dass der Nerv durch das Fräsen verletzt wird.

Erfindungsgemäß sieht die Erfindung zur Lösung der genannten Aufgabe einen gattungsgemäßen Facettengelenkfräser mit den kennzeichnenden Merkmalen des Anspruchs 1 vor.

Ein erfindungsgemäßer Facettengelenkfräser ist gekennzeichnet durch eine die Zähnung axial überragende, sich über einen Teilumfang erstreckende Lippe, wobei der Erstreckungsbogen- oder Winkel zwischen 140 und 160°, vorzugsweise 150° liegt. Die axiale Höhe der Lippe liegt vorzugsweise in einem Bereich von 1 bis 3 mm.

Die Lippe ist dabei entgratet und an den Kanten verrundet. Sie dient bei nervennahem Arbeiten mit dem Fräser als Schutz für den Nerv, so dass dieser durch das Fräsen nicht verletzt wird. Zur Orientierung ist das proximale, also der Zähnung abgewandte Ende des Fräsers entsprechend der Position der Lippe mit einer Markierung, beispielsweise einer erhöhten Rippe, versehen.

Insbesondere bei einem mit einer solchen Lippe ausgebildeten Fräser wird dieser nicht vollständig gedreht, sondern oszillierend hin und her gedreht, so dass die Zähnung immer im Eingriff mit dem Knochenmaterial steht.

Durch die Aufweitung des distalen Endes des Facettengelenkfräsers, insbesondere über die Höhe der Zähnung des Fräsers, vorzugsweise aber bis etwa zum Zweifachen der Höhe der Zähnung des Fräsers hin wird das Eintreten von abgetragenem Knochenmaterial in das Fräserinnere verbessert, worauf dieses dann gegebenenfalls angesaugt werden kann. Die Aufweitung erstreckt sich stetig und kontinuierlich von dem Querschnitt des Schaftes des Fräsers bis zur maximalen Aufweitung an der Stirnseite der Zähnung und beträgt zwischen 0,2 und 0,6, vorzugsweise 0,4 mm, so dass also der Durchmesser des Fräsers an der Stirnseite der Zähnung zwischen 0,2 und 0,6, vorzugsweise 0,4 mm über dem Durchmesser des zylindrischen Schaftes liegt.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnungen im Einzelnen erläutert ist. Dabei zeigt:
- Fig. 1: einen Fräser in Seitenansicht;
- Fig. 2: eine vergrößerte Darstellung des distalen oder Kopf-Endes des Fräsers der Fig. 1;
- Fig. 3: einen Blick auf die distale Stirnseite des Fräsers der Fig. 2 entsprechend dem Pfeil III in der Fig. 2;
- Fig. 4: eine Seitenansicht einer besonders bevorzugten Ausgestaltung eines erfindungsgemäßen Fräsers;
- Fig. 5: das distale Ende des Fräsers der Fig. 4 in vergrößerter Darstellung;
- Fig. 6: eine Stirnansicht entsprechend VI der Fig. 4 auf diesen Fräser;
- Fig. 7: ein vorderes Ende eines erfindungsgemäßen Fräsers mit aus Raspel ausgebildeter Lippe;
- Fig. 8: eine Rückansicht zweier benachbarter Lendenwirbel der menschlichen Wirbelsäule; und
- Fig. 9: eine teilweise geschnitten dargestellte Ansicht der spinalen Bandscheiben zwischen den beiden Wirbeln gemäß Fig. 7.

Der erfindungsgemäße Facettengelenkfräser weist einen Schaft 1a auf, der vorzugsweise eine Länge im Bereich zwischen 18 und 25 cm hat, im dargestellten Ausführungsbeispiel etwa 21 cm. An seinem rückwärtigen oder - zum Operateur hin - proximalen Ende 2 ist der Fräser 1 entweder mit einem Griff versehen oder mit einer Befestigungseinrichtung zur lösbaren Befestigung an einem Griff, die letztere vorzugsweise aus Kunststoff besteht. Weder Befestigungseinrichtung noch Griff sind im Detail dargestellt.

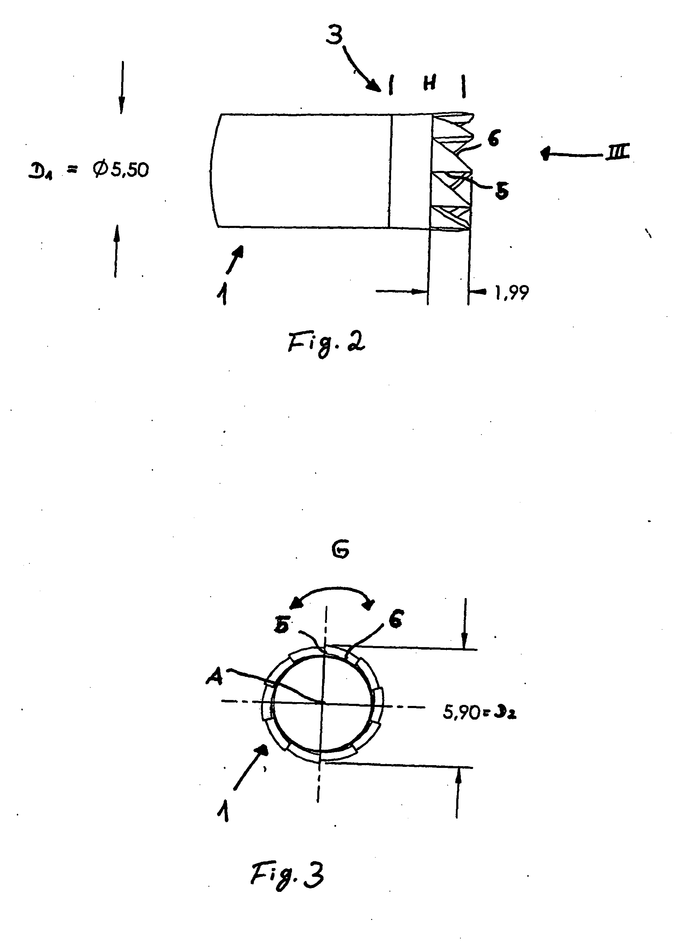

An seinem vorderen oder distalen Ende 3 befindet sich eine Zähnung 4. Im dargestellten Ausführungsbeispiel ist die Zähnung 4 asymmetrisch ausgebildet. Bei Blick auf die Stirnseite entsprechend der Fig. 3 verläuft gegen den Uhrzeigersinn G gesehen die vordere Flanke 5, parallel zur Achse A, während die rückwärtige Flanke 6 eine abgeschrägte Flanke mit einem Neigungswinkel von 35 bis 45°, vorzugswei-, se ca. 40° zur Achse A ist (Die vordere Flanke 5 ist beim Arbeiten eine in Drehrichtung die aktive fräsende Flanke) .

Wie weiterhin insbesondere aus den Darstellungen der Fig. 2 und 3 entnehmbar ist, kann das stirnseitige Ende des Fräsers 1 gegenüber dem Durchmesser des Schaftes 1a aufgeweitet sein. Während im dargestellten Ausführungsbeispiel der Schaft einen Durchmesser von 5,5 mm hat, weist die distale Stirnseite des Fräsers im Bereich der Zähnung 4 einen Durchmesser von 5,9 mm auf. Es ist also eine Aufweitung von 0,4-mm gegeben. Vorzugsweise liegt die Aufweitung im Bereich von etwa 0,8 mm bzw. 2 % des Rohrdurchmessers bis 0,6 mm mit einer Toleranz von 0,01 mm. Der Durchmesser D₁ des Schafts 1a bis zum Durchmesser D₂ der Stirnseite der Zähnung 4 erstreckt sich über eine Höhe H, also knapp 2 mm, so dass die Höhe H des Aufweitebereichs etwa 4 mm beträgt, und vorzugsweise bis zu 6 mm betragen kann.

Durch die Aufweitung wird erreicht, dass abgetragenes Knochenmaterial in das Innere des Fräsers eintritt und so aus dem Arbeitsbereich und damit dem Körper des Patienten entfernt werden kann, beispielsweise durch Absaugen durch das Fräserinnere.

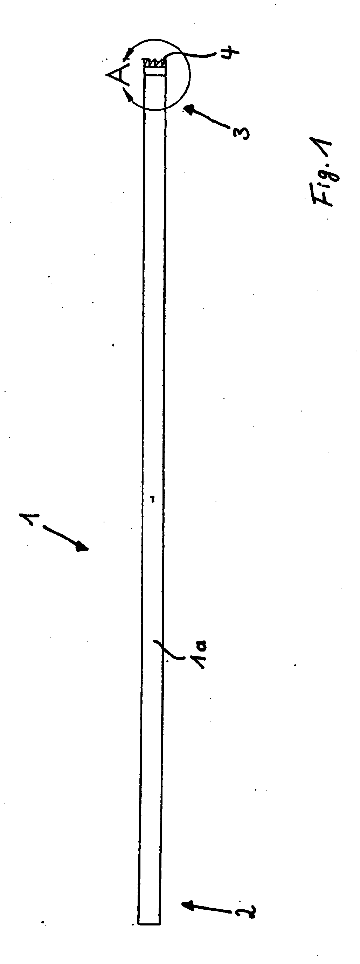

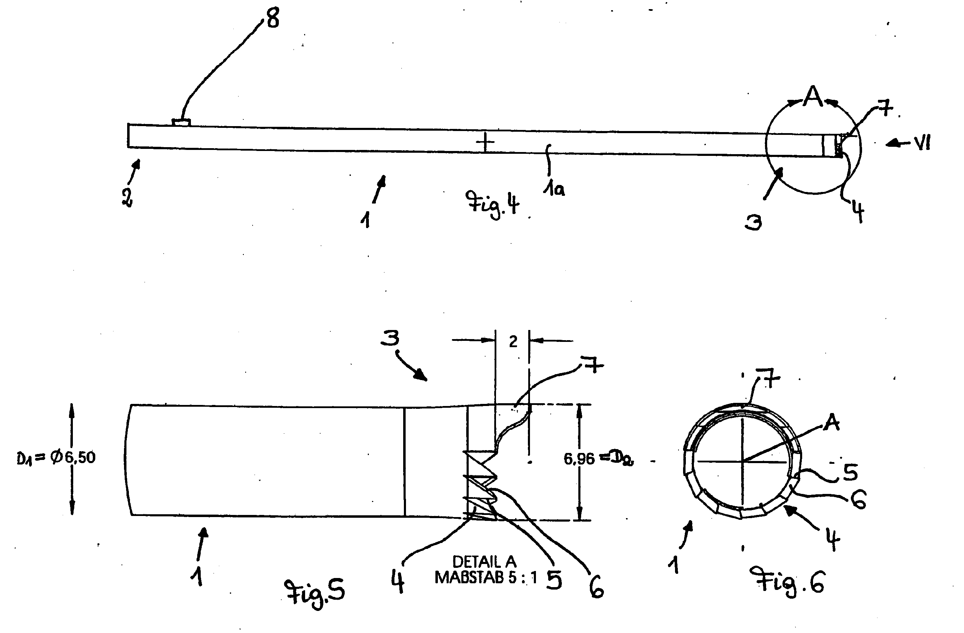

Die Fig. 4 bis 6 zeigen eine äußerst bevorzugte Ausgestaltung eines erfindungsgemäßen Fräsers. Soweit gleiche Teile vorhanden sind, so werden gleiche Bezugszeichen verwendet; auf die Ausgestaltung wird insofern auch nicht mehr eingegangen, sondern hierzu auf die vorstehende Erläuterung zu den Fig. 1 bis 3 verwiesen.

Abweichend von dem Fräser der Fig. 1 bis 3 weist der erfindungsgemäße Fräser 1 der Fig. 4 bis 6 eine die Zähnung axial überragende, sich über einen Teilumfang oder Teilbogen des Schaftmantels erstreckende Lippe 7 auf. Die bogenförmige Erstreckung reicht über einen Winkel von im dargestellten Ausführungsbeispiel von 150°, die Höhe der Lippe 7 über die Zähnungsspitzen hinaus beträgt - unabhängig vom Durchmesser des Fräsers - ca. 2 mm. Die Lippe ist entgrated und die Kanten sind verrundet. Durch die Lippe 7 wird bei nervennahem Arbeiten ein benachbarter Nerv geschützt, so dass dieser durch das Fräsen nicht verletzt wird.

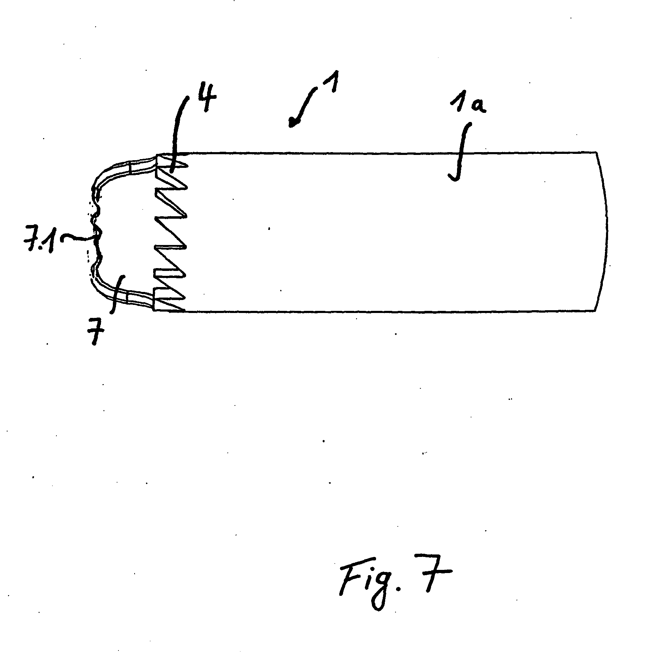

Bei der Ausgestaltung der Fig. 7 ist die Stirnseite der Lippe 7 als Raspel 7.1 mit einer wellenartigen Zähnung ausgebildet. Hierdurch können bei Schwenkbewegungen unter Druck weiche Gewebeteile, wie Knochenhaut (Perios) entfernt werden; während die härtere Nervenhaut durch einen solchen Raspel 7.1 nicht beschädigt wird.

Zur Orientierung ist das proximale Ende 2 des Fräsers 1 mit einer Markierung 8 versehen, die der Position der Lippe 7 entspricht, also in axialer Richtung mit dieser fluchtet.

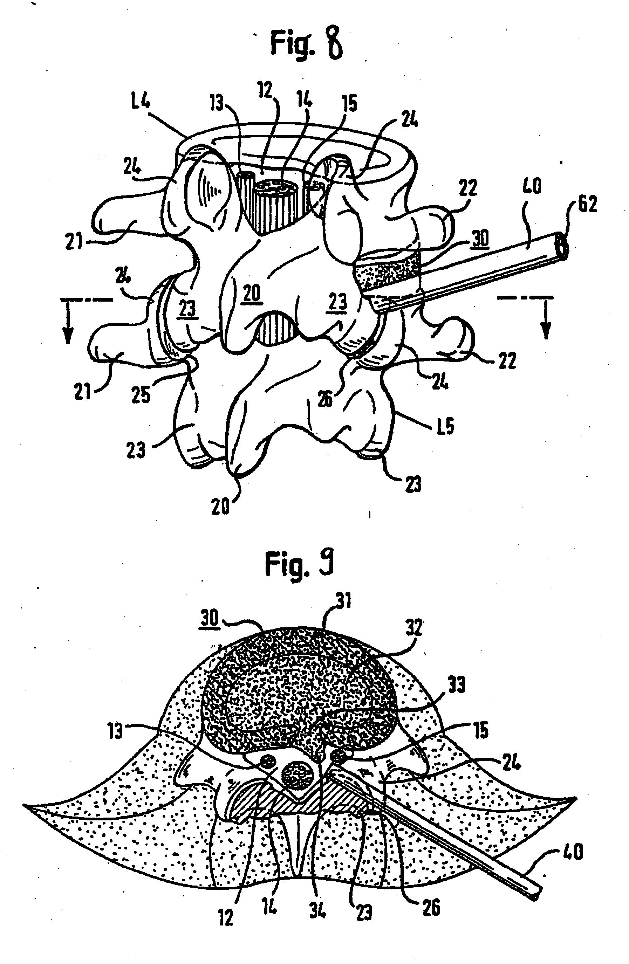

Der erfindungsgemäße Fräser wird in der im Folgenden unter Bezug auf die Fig. 8 und 9 beschriebenen Weise eingesetzt.

Die Fig. 8 zeigt beispielhaft den vierten und fünften Lendenwirbel L4, L5 und zwischen diesen eine Bandscheibe 30 mit einer Einklemmung 33 am Faserknorpelring 31 (Annulus fibrosus) unmittelbar zur Rechten der Mittelachse der Wirbelsäule mit einer Extrusion 34 von Nucleus pulposus-Gewebe in den Canalis vertebralis - Fig. 5.

Im Inneren des Wirbelkanals 12 (Canalis vertebralis) sind schematisch Nervenstrukturen 13, 14, 15 dargestellt. Jeder Wirbel L4, L5 weist einen Dornfortsatz 20 und einen linken und rechten Querfortsatz 22, linke und rechte untere, ein Gelenk bildende Fortsätze 23 sowie linke und rechte obere, ein Gelenk bildende Fortsätze 24 auf, wobei das linke und das rechte Gelenk zwischen dem oberen und dem unteren Lendenwirbel L4, L5, das als Facettengelenk 26 bezeichnet wird, durch jeweils die unteren Fortsätze 23 des oberen Wirbels L4 und die oberen Fortsätze 24 des unteren Wirbel L5 gebildet ist.

Das Arbeiten mit dem erfindungsgemäßen Fräser gestaltet sich in der folgenden Weise:

Zunächst wird eine Hohlnadel mit einem Außendurchmesser in der Größenordnung von 1,25 mm in eine der Einklemmung benachbarten Position vorgeschoben. Sodann wird ein Führuhgsdraht durch das Lumen geschoben, bis sein distales Ende das Ende der Hohlnadel etwas überragt. Anschließend wird die Hohlnadel entnommen, wogegen der Führungsdraht am Ort verbleibt. Es wird sodann ein Führungsstab mit Außendurchmesser 2,5 mm (ausgebildet auch als Dilatator) über den Führungsdraht vorgeschoben, bis dann das konische Ende des Führungsstabes (AD 2,5 mm) sich an dem Facettengelenk 26 befindet. Über den Führungsstab wird nun noch eine Führungshülse (AD 3,8 mm), am distalen Ende konisch, zur weiteren Gewebedilatation und zur Fräserführung geschoben. Unter Halten des Führungsstabs mit Führungshülse in dieser Position wird ein erfindungsgemäßer Fräser mit einem geringen Durchmesser in der Größenordnung von Innendurchmesser 4,2 mm, Außendurchmesser 5,0 mm über den Führungsstab und die Führungshülse 52 vorgeschoben, bis das distale Ende des Fräsers an der Oberfläche des Facettengelenks 26 zur Anlage kommt.

Nun dreht der Operateur an dem ihm nahen (proximalen) Ende des Fräsers den dort vorgesehenen Handgriff, beispielsweise von Hand, vorzugsweise oszillierend, so dass im Vorsprung 23 des Wirbels L4 ein Kanal erzeugt wird. Dieser Schritt wird mit Führungsstäben, Führungshülsen und Fräsern größeren Durchmessers wiederholt, bis ein hinreichender Durchmesser des Kanals erreicht ist, um eine Arbeitskanüle mit einem Lumen aufzunehmen, das hinreichend groß ist, um nicht nur ein Zangenwerkzeug, sondern auch ein Endoskop hindurchführen zu können. Anschließend wird mit dem Zangenwerkzeug, gegebenenfalls unter Endoskopbeobachtung, die Einklemmung entfernt.

### Bezugszeichenliste

- 1: Fräser
- 1a: Schaft
- 2: proximales Ende
- 3: distales Ende
- 4: Zähnung
- 5: vordere Flanke
- 6: rückwärtige Flanke
- 7: Lippe
- 7.1: Raspel
- 12: Wirbelkanal
- 13, 14, 15: Nervenstrukturen
- 20: Dornfortsatz
- 22: Querfortsatz
- 23, 24: Fortsätze
- 26: Facettengelenk
- 30: Bandscheibe
- 31: Faserknorpelring
- 33: Einklemmung
- 34: Extrusion
- 54: Trokar

- A: Achse

- D₁, D₂: Durchmesser
- G: Gegenuhrzeigersinn ( 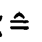 Drehrichtung)
- H: Höhe
- L4, L5: Lendenwirbel

## Patentansprüche

1. Facettengelenkfräser mit einem Schaft (1a) und einer Zähnung (4) an der distalen Stirnseite des Schaftes, **dadurch gekennzeichnet, dass** die distale Stirnseite des Schaftes eine die Zähnung axial überragende, sich über einen Teilumfang erstreckende Lippe (7) aufweist.

2. Fräser nach Anspruch 1, **dadurch gekennzeichnet, dass** die Lippe die Zähnung 1 bis 3 mm überragt.

3. Fräser nach Anspruch 1 oder 2, **dadurch gekennzeichnet, dass** die Lippe sich über einen Bogen von 140 bis 160°, vorzugsweise von 150°, erstreckt.

4. Fräser nach einem der Ansprüche 1 bis 3, **dadurch gekennzeichnet**, das zumindest die Stirnseite der Lippe (7) als Raspel (7.1) ausgebildet ist.

5. Fräser nach einem der Ansprüche 1 bis 4, **dadurch gekennzeichnet, dass** die distale Stirnseite des Fräsers gegenüber dem Durchmesser des Schafts am proximalen Ende aufgeweitet ist.

6. Fräser nach Anspruch 5, **dadurch gekennzeichnet, dass** die Aufweitung der Stirnseite des Fräsers sich mindestens über die Höhe der Zähnung erstreckt.

7. Fräser nach Anspruch 5 oder 6, **dadurch gekennzeichnet, dass** die Aufweitung sich etwa über das Doppelte der Höhe der Zähnung erstreckt.

8. Fräser nach einem der Ansprüche 5 bis 7, **dadurch gekennzeichnet, dass** die Aufweitung kontinuierlich stetig vom Durchmesser des Schafts auf den Maximaldurchmesser der Aufweitung an den Spitzen der Zähnung verläuft.

9. Fräser nach einem der Ansprüche 5 bis 8, **dadurch gekennzeichnet, dass** die Stirnseite im Bereich der Spitzen der Zähnung zwischen 2 und 5 mm aufgeweitet ist.

10. Fräser nach einem der Ansprüche 5 bis 9, **dadurch gekennzeichnet, dass** die radiale Aufweitung an den Spitzen der Zähnung etwa 0,4 mm beträgt.

## Claims

1. A facet joint milling cutter having a shank (1a) and teeth (4) at the distal end of the shank, **characterised in that** the distal end of the shank comprises a lip (7) projecting axially beyond the teeth and extending over a part of the circumference.

2. A milling cutter according to claim 1, **characterised in that** the lip projects beyond the teeth by 1 to 3 mm.

3. A milling cutter according to claim 1 or claim 2, **characterised in that** the lip extends over an arc of 140 to 160°, preferably 150°.

4. A milling cutter according to any one of claims 1 to 3, **characterised in that** at least the end of the lip (7) takes the form of a rasp (7.1).

5. A milling cutter according to any one of claims 1 to 4, **characterised in that** the distal end of the milling cutter is widened relative to the diameter of the shank at the proximal end.

6. A milling cutter according to claim 5, **characterised in that** the widened portion of the end of the milling cutter extends at least over the height of the teeth.

7. A milling cutter according to claim 5 or claim 6, **characterised in that** the widened portion extends approximately over twice the height of the teeth.

8. A milling cutter according to any one of claims 5 to 7, **characterised in that** widening proceeds continuously and steadily from the diameter of the shank to the maximum diameter of the widened portion at the tips of the teeth.

9. A milling cutter according to any one of claims 5 to 8, **characterised in that** the end is widened over between 2 and 5 mm in the area of the tips of the teeth.

10. A milling cutter according to any one of claims 5 to 9, **characterised in that** the radial widening at the tips of the teeth amounts to approximately 0.4 mm.

## Revendications

1. Fraise pour articulation à facettes avec une queue (1a) et une dentelure (4) à la face frontale distale de la queue, **caractérisée en ce que** la face frontale distale de la queue présente une lèvre (7) dépassant axialement de la dentelure et s'étendant sur une partie de la circonférence.

2. Fraise selon la revendication 1, **caractérisée en ce que** la lèvre dépasse de la dentelure de 1 à 3 mm.

3. Fraise selon les revendications 1 ou 2, **caractérisée en ce que** la lèvre s'étend sur un arc de 140 à 160°, et de préférence de 150°.

4. Fraise selon une des revendications 1 à 3, **caractérisée en ce qu'**au moins la face frontale de la lèvre (7) est réalisée en forme de râpe (7.1).

5. Fraise selon une des revendications 1 à 4, **caractérisée en ce que** la face frontale distale de la fraise est évasée par rapport au diamètre de la queue à l'extrémité proximale.

6. Fraise selon la revendication 5, **caractérisée en ce que** l'évasement de la face frontale de la fraise s'étend au moins au dessus de la hauteur de la dentelure.

7. Fraise selon une des revendications 5 ou 6, **caractérisée en ce que** l'évasement s'étend au double de la hauteur de la dentelure.

8. Fraise selon une des revendications 5 à 7, **caractérisée en ce que** l'évasement progresse régulièrement du diamètre de la queue au diamètre maximal de l'évasement aux pointes de la dentelure.

9. Fraise selon une des revendications 5 à 8, **caractérisée en ce que** la face frontale est évasée de 2 à 5 mm au niveau des pointes de la dentelure.

10. Fraise selon une des revendications 5 à 9, **caractérisée en ce que** l'évasement radial est d'environ 0,4 mm aux pointes de la dentelure.
